# EUROPÄISCHE PATENTSCHRIFT

(11) **EP 2 406 212 B1**
(45) Veröffentlichungstag und Bekanntmachung des Hinweises auf die Patenterteilung: **15.05.2013**
(21) Anmeldenummer: 10707540.0
(22) Anmeldetag: 11.03.2010
(51) Int. Cl.: C07C 209/22

(54) **VERFAHREN ZUR HERSTELLUNG VON 1-ADAMANTYLTRIMETHYLAMMONIUMHYDROXID**
METHOD FOR PRODUCING 1-ADAMANTYL TRIMETHYLAMMONIUM HYDROXIDE
PROCÉDÉ POUR PRODUIRE DE L'HYDROXYDE DE 1-ADAMANTYL-TRIMÉTHYLAMMONIUM

(30) Priorität: 12.03.2009 EP 09155016
(43) Veröffentlichungstag der Anmeldung: 18.01.2012
(73) Patentinhaber: BASF SE, 67056 Ludwigshafen (DE)
(72) Erfinder: WIGBERS, Christof, Wilhelm, 68167 Mannheim (DE); ERNST, Martin, 69115 Heidelberg (DE); SZARVAS, Laszlo, 67071 Ludwigshafen (DE); D'ANDOLA, Giovanni, 69120 Heidelberg (DE); DAHLHOFF, Ellen, 67117 Limburgerhof (DE); IFFLAND, Gabriele, 69126 Heidelberg (DE); RAATZ, Peter, 67069 Ludwigshafen (DE); SIMON, Falk, 64625 Bensheim (DE); FRAUENKRON, Matthias, 67251 Freinsheim (DE)
(86) Internationale Anmeldenummer: PCT/EP2010/053077
(87) Internationale Veröffentlichungsnummer: WO 2010/103062

(56) Entgegenhaltungen:
- EP-A1- 0 139 504
- EP-A2- 0 231 018
- WO-A1-2005/115969
- WO-A2-2005/085207
- E.V.VASHKEVICH ET AL: "Synthesis of surfactants derived from adamantane" RUSSIAN JOURNAL OF APPLIED CHEMISTRY, Bd. 74, Nr. 11, 2001, Seiten 1892-1898, XP002578328

## Beschreibung

Die vorliegende Erfindung betrifft ein Verfahren zur Herstellung von 1-Adamantyltrimethylammoniumhydroxid.

1-Adamantyltrimethylammoniumhydroxid ist z. B. von Interesse für die Herstellung von Katalysatoren, beispielsweise als Templat bei der Synthese von Zeolithen, die ihrerseits als Katalysator(komponente) verwendet werden können. Es besteht daher ein Bedarf an wirtschaftlichen Wegen zu seiner Herstellung.

Zur Herstellung von Ammoniumsalzen organischer Amine ist es seit langem bekannt, Amine mit Dialkylsulfaten zu alkylieren, wobei in der Regel jedoch nur eine Alkylgruppe des Dialkylsulfats ausgenutzt wird, so dass die entsprechenden Monoalkylsulfatsalze resultieren.

Die WO 2005/085207 beschreibt ein Verfahren zur Herstellung von ionischen Verbindungen, die Kationen mit quartären sp²-hybridisierten Stickstoffatomen umfassen, bei dem man Verbindungen, die ein doppelt gebundenes Stickstoffatom enthalten, mit einem Dialkylsulfat bei erhöhter Temperatur und unter Einsatz beider Alkylgruppen des Dialkylsulfats umsetzt, wobei eine ionische Verbindung mit Sulfatanionen erhalten wird und diese gegebenenfalls einem Anionenaustausch unterzieht. Der Anionenaustausch kann dabei durch Umprotonierung, Umsetzung mit einem Metallsalz, lonenaustauschchromatographie, elektrolytisch oder durch eine Kombination dieser Maßnahmen erfolgen.

Die WO 2005/115969 betrifft ein Verfahren zur Herstellung von quartären AmmoniumVerbindungen, bei dem man eine Aminverbindung, die wenigstens ein sp³-hybridisiertes Stickstoffatom enthält, mit einem Dialkylsulfat unter Einsatz beider Alkylgruppen des Dialkylsulfats umsetzt, wobei eine quartäre Ammoniumverbindung mit Sulfatanionen erhalten wird und anschließend das Sulfatanion gegen ein davon verschiedenes Anion austauscht. Der Anionenaustausch kann wie in der WO 2005/085207 beschrieben erfolgen.

Die BE 646581 beschreibt Derivate des Adamantans, verschiedene Verfahren zu ihrer Herstellung und ihre medizinische Verwendung, z. B. als Antivirusmittel. So ist beispielsweise die Alkylierung von 1-Aminoadamantan mit Methyliodid zu 1-Adamantyltrimethylammoniumiodid, die weitere Umsetzung mit Aminoethanol zu 1-Adamantyldimethylamin und dessen Isolierung als Säuresalz der Perchlorsäure beschrieben. Nachteilig am Einsatz von Methyliodid zur Alkylierung ist, dass immer Produktgemische mit unterschiedlichen Alkylierungsgraden erhalten werden und dessen hoher Preis.

Die EP 0 139 504 A1 beschreibt ein Verfahren zur katalytischen Paraffin-OlefinAlkylierung unter Einsatz eines Katalysators, der wenigstens ein Adamantanderivat enthält. Als geeignete Adamantanderivate sind auch 1-Adamantyltrimethylammoniumsalze genannt, als bevorzugtes Anion ist das Sulfat erwähnt.

Die EP 0 231 018 A2 beschreibt einen kristallinen Zeolith und dessen Herstellung unter Verwendung von quartären Ammoniumverbindungen des Adamantans. Das Beispiel 1 dieses Dokuments beschreibt die Herstellung von 1-Adamantyltrimethylammoniumhydroxid durch Alkylierung von 1-Aminoadamantan mit Methyliodid und anschließenden Austausch der lodidanionen an einem mit OH-Ionen beladenen Ionenaustauscher. Ein ähnliches Verfahren wird in dem Dokument Russian Journal of Applied chemistry, 74 (11), 2001, 1892-1898 erwähnt. Überraschenderweise wurde gefunden, dass sich 1-Adamantyltrimethylammoniumhydroxid in vorteilhafter Weise herstellen lässt, wenn man von 1-Adamantyldimethylamin ausgeht, dieses einer Alkylierung mit Dimethylsulfat unterzieht und das Alkylierungsprodukt anschließend einem Anionenaustausch an einem mit OH-Ionen beladenen Ionenaustauscher unterzieht. Dabei gelingt es bei der Alkylierung mit Dimethylsulfat, im Wesentlichen das 1-Adamantyltrimethylammoniumsalz des Sulfats (SO₄²⁻) und nicht des Methylsulfats (Methosulfats, CH₃OSO₃⁻) zu erhalten. Das 1-Adamantyltrimethylammoniumsalz des Sulfats zeichnet sich durch seine hohe Affinität zu lonenaustauscherharzen aus und lässt sich besser einem Anionenaustausch unterziehen als das 1-Adamantyltrimethylammoniumsalz des Methylsulfats.

Gegenstand der Erfindung ist daher ein Verfahren zur Herstellung von 1-Adamantyltrimethylammoniumhydroxid, bei dem man
A) 1-Adamantyldimethylamin bereitstellt und einer Umsetzung mit Dimethylsulfat unterzieht, wobei im Wesentlichen beide Methylgruppen des Dimethylsulfats abreagieren und im Wesentlichen 1-Adamantyltrimethylammoniumsulfat erhalten wird, und
B) das in Schritt A) erhaltene 1-Adamantyltrimethylammoniumsulfat einem Anionenaustausch an einem mit OH-Ionen beladenen Ionenaustauscher unterzieht.

### Schritt A)

Zur Bereitstellung des 1-Adamantyldimethylamins in Schritt A) kann man 1-Adamantylamin oder ein 1-Adamantylammoniumsalz einer Alkylierung im Sinne einer Dimethylierung unterziehen. Im Wesentlichen reagieren beide Methylgruppen des Dimethylsulfats ab. Unter dem Begriff, dass "im Wesentlichen beide Methylgruppen des Dimethylsulfats abreagieren" versteht man im Rahmen der vorliegenden Erfindung, dass wenigstens 90 %, vorzugsweise wenigstens 95 % der beiden Methylgruppen des Dimethylsulfats abreagieren. Dementsprechend enthält das bei der Reaktion erhaltene Methylierungsprodukt höchstens 10 Gew.-%, vorzugsweise höchstens 5 Gew.-%, insbesondere höchstens 1 Gew.-% an Methosulfat, CH₃OSO₃⁻. In einer speziellen Ausführungsform liegt der Gehalt an Methosulfat deutlich unter 1 Gew.-%, speziell unter 0,5 Gew.-% oder unter 0,1 Gew.-%.

Vorzugsweise geht man zur Bereitstellung des 1-Adamantyldimethylamins von 1-Adamantylammoniumhydrochlorid aus.

In einer speziellen Ausführung des erfindungsgemäßen Verfahrens wird das 1-Adamantylammoniumhydrochlorid zunächst in 1-Adamantylamin überführt. Dazu kann das 1-Adamantylammoniumhydrochlorid mit wässriger Base umgesetzt werden. Geeignete Basen sind z. B. Alkalihydroxide, wie NaOH oder KOH.

Vorzugsweise erfolgt die Umsetzung des 1-Adamantylammoniumhydrochlorids mit wässriger Base in Gegenwart eines organischen Lösungsmittels, das nicht wassermischbar ist und in dem sich das gebildete 1-Adamantylamin löst. Unter einem nicht wassermischbaren organischen Lösungsmittel wird im Rahmen der vorliegenden Erfindung ein Lösungsmittel verstanden, das sich unter Normalbedingungen (20 °C, 1013 mbar) zu höchstens 10 g in 100 g Wasser löst und bei dem sich unter Normalbedingungen höchstens 10 g Wasser in 100 g Lösungsmittel lösen. Geeignete organische Lösungsmittel sind aromatische Kohlenwasserstoffe, wie Benzol, Toluol, Ethylbenzol oder Xylol; halogenierte Lösungsmittel, wie Dichlormethan, Chloroform, Tetrachlorkohlenstoff, Dichlorethan oder Chlorbenzol; aliphatischen Lösungsmittel, wie Pentan, Hexan, Heptan, Octan, Ligroin, Petrolether, Cyclohexan oder Dekalin; und Mischungen davon. Bevorzugt sind Toluol oder Xylol. Die Umsetzung erfolgt dann im Sinne einer Extraktivreaktion, wobei das freigesetzte Amin im Wesentlichen vollständig in die organische Phase übergeht. Zur Isolierung des 1-Adamantylamins kann das organische Lösungsmittel nach üblichen Verfahren, z. B. Verdampfen, entfernt werden. Das Verdampfen des Lösungsmittels kann in dafür üblichen Vorrichtungen erfolgen. Das Verdampfen des Lösungsmittels erfolgt vorzugsweise unter vermindertem Druck und/oder erhöhter Temperatur. Bevorzugt wird das 1-Adamantylamin als Lösung in dem organischen Lösungsmittel zur weiteren Umsetzung eingesetzt.

In einer ersten Variante des erfindungsgemäßen Verfahrens wird zur Bereitstellung des 1-Adamantyldimethylamins in Schritt A) Formaldehyd einer reduktiven Aminierung mit 1-Adamantylamin oder einem 1-Adamantylammoniumsalz und mit Wasserstoff in Gegenwart eines Hydrierkatalysators unterzogen.

Wird zur reduktiven Aminierung nach der ersten Variante ein 1-Adamantylammoniumsalz eingesetzt, so handelt es sich vorzugsweise um 1-Adamantylammoniumhydrochlorid. Alternativ dazu wird zur reduktiven Aminierung nach der ersten Variante 1-Adamantylamin eingesetzt, das durch vorherige Umsetzung von 1-Adamantylammoniumhydrochlorid mit einer wässrigen Base, wie zuvor beschrieben, erhältlich ist.

Zur reduktiven Aminierung nach der ersten Variante wird ein unter den Reaktionsbedingungen inertes Lösungsmittel oder Lösungsmittelgemisch eingesetzt. Geeignet sind Wasser, organische Lösungsmittel und Mischungen davon. Geeignet sind weiterhin Kombinationen aus teilweise oder vollständig nicht miteinander mischbaren Lösungsmitteln. In einer bevorzugten Ausführung erfolgt die Umsetzung in einem System, das zwei flüssige Phasen aufweist. Geeignete organische Lösungsmittel sind vorzugsweise ausgewählt unter Alkoholen, wie Methanol, Ethanol, n-Propanol, Isopropanol, n-Butanol, tert.-Butanol oder Phenol, Di- und Polyolen, wie Ethandiol und Propandiol, aromatischen Kohlenwasserstoffen, wie Benzol, Toluol, Ethylbenzol oder Xylolen, halogenierten Lösungsmitteln, wie Dichlormethan, Chloroform, Tetrachlorkohlenstoff, Dichlorethan oder Chlorbenzol, aliphatischen Lösungsmittel, wie Pentan, Hexan, Heptan, Octan, Ligroin, Petrolether, Cyclohexan oder Dekalin, Ethern, wie Tetrahydrofuran, Diethylether, Methyl-tert.-Butylether oder Diethylenglycolmonomethylether, und Mischungen davon. Bevorzugt wird zur reduktiven Aminierung ein Gemisch aus Wasser und wenigstens einem nicht wassermischbaren organischen Lösungsmittel eingesetzt. Bevorzugt ist ein Gemisch, das Wasser und wenigstens einen aromatischen Kohlenwasserstoff, wie Benzol, Toluol, Ethylbenzol oder Xylol, enthält. In einer speziellen Ausführung wird ein Wasser/Xylol-Gemisch eingesetzt.

Die Reaktionstemperatur liegt vorzugsweise in einem Bereich von 20 bis 250 °C, insbesondere von 50 bis 200 °C.

Der Druck liegt vorzugsweise in einem Bereich von 1 bis 300 bar, besonders bevorzugt von 5 bis 200 bar, insbesondere von 10 bis 150 bar.

Der Formaldehyd wird vorzugsweise in Form einer wässrigen Lösung eingesetzt. Der Formaldehydgehalt der wässrigen Lösung liegt vorzugsweise in einem Bereich von etwa 10 bis 40 %, vorzugsweise etwa 20 bis 35 % (je nach Bedingungen weisen gesättigte, wässrige Lösung von Formaldehyd einen Formaldehydgehalt von 36 bis 40 % auf).

Zur reduktiven Aminierung nach der ersten Variante unter Verwendung von Wasser oder wasserhaltigen Lösungsmittelgemischen kann der wässrigen Phase ein Puffer zur Einstellung des pH-Werts zugesetzt werden. Vorzugsweise wird der Puffer so ausgewählt, dass der pH-Wert der wässrigen Phase während der Umsetzung in einem Bereich von etwa 5 bis 10 und vorzugsweise etwa 7 bis 10 liegt. Bevorzugt als Puffersubstanz ist NaH₂PO₄. Der Zusatz von NaH₂PO₄ kann auch durch seine Befähigung zur Verhinderung von Korrosion durch die Bildung korrosionshemmender Deckschichten vorteilhaft sein.

Das Molmengenverhältnis von Formaldehyd zu 1-Adamantylamin (oder dem 1-Adamantylammoniumsalz) liegt bevorzugt in einem Bereich von 1,8 : 1 bis 10 : 1, besonders bevorzugt von 2,0 : 1 bis 5 : 1, insbesondere 2,01 : 1 bis 3 : 1.

Als Hydrierkatalysator können handelsübliche Katalysatoren verwendet werden. Insbesondere geeignet sind Katalysatoren, die Palladium, Rhodium, Ruthenium, Platin, Eisen, Cobalt, Kupfer, Nickel als hydrieraktive Metalle enthalten.

Als Hydrierkatalysator wird vorzugsweise ein Raney-Metall, besonders bevorzugt Raney-Nickel, eingesetzt.

Als Hydrierkatalysator eignen sich bevorzugt auch solche Katalysatoren, die als Aktivkomponente Palladium enthalten. Neben Palladium kann der Katalysator auch weitere Aktivkomponenten, die vorzugsweise ausgewählt sind unter Zink, Cadmium, Platin, Silber, Seltenerdmetallen und Mischungen davon enthalten. Ein geeignetes Seltenerdmetall ist Cer.

Das nach der ersten Variante des erfindungsgemäßen Verfahrens erhaltene Produkt der reduktiven Aminierung kann vor seiner weiteren Umsetzung mit Dimethylsulfat einer Aufarbeitung unterzogen werde. Bevorzug ist eine destillative Aufarbeitung.

Eine bevorzugte Ausführung ist ein Verfahren, bei dem man
a) 1-Adamantylammoniumhydrochlorid durch Umsetzung mit einer wässrigen Lösung einer Base in das 1-Adamantylamin überführt,
b) das 1-Adamantylamin einer reduktiven Aminierung mit Formaldehyd und Wasserstoff in Gegenwart eines Hydrierkatalysators zu 1-Adamantyldimethylamin unterzieht,
c) das 1-Adamantyldimethylamin einer Umsetzung mit Dimethylsulfat unterzieht, wobei im Wesentlichen beide Methylgruppen des Dimethylsulfats abreagieren und im Wesentlichen 1-Adamantyltrimethylammoniumsulfat erhalten wird, und
d) das 1-Adamantyltrimethylammoniumsulfat einem Anionenaustausch an einem mit OH-Ionen beladenen Ionenaustauscher unterzieht.

Bezüglich der Schritte a) und b) wird auf die vorherigen Ausführungen zur Überführung von 1-Adamantylammoniumhydrochlorid in 1-Adamantylamin und die katalytische reduktive Aminierung mit Formaldehyd und Wasserstoff in vollem Umfang Bezug genommen. Bezüglich der Schritte c) und d) wird auf die folgenden Ausführungen zur Umsetzung des 1-Adamantyldimethylamins mit Dimethylsulfat und den Anionenaustausch an einem mit OH-Ionen beladenen Ionenaustauscher (= Schritt B) in vollem Umfang Bezug genommen.

In einer zweiten Variante des erfindungsgemäßen Verfahrens wird zur Bereitstellung des 1-Adamantyldimethylamins in Schritt A) Formaldehyd einer reduktiven Aminierung mit 1-Adamantylamin oder einem 1-Adamantylammoniumsalz in Gegenwart von Ameisensäure unterzogen. Die reduktive Alkylaminierung von Carbonylverbindungen in Gegenwart von Ameisensäure ist unter der Bezeichnung Leuckart-Reaktion literaturbekannt. Die reduktive Aminmethylierung mit Formaldehyd als Carbonylkomponente in Gegenwart von Ameisensäure wird als Eschweiler-Clark-Reaktion bezeichnet. Geeignete Reaktionsbedingungen finden sich z. B. in J. March, Advanced Organic Chemistry, Verlag John Wiley & Sons, 4. Auflage, S. 898 bis 900 und der dort zitierten Literatur, worauf hier in vollem Umfang Bezug genommen wird.

Zur reduktiven Aminierung nach der zweiten Variante wird vorzugsweise 1-Adamantylammoniumhydrochlorid eingesetzt. Es ist jedoch auch möglich, zur reduktiven Aminierung nach der zweiten Variante von 1-Adamantylamin auszugehen.

Zur reduktiven Aminierung nach der zweiten Variante kann ein unter den Reaktionsbedingungen inertes Lösungsmittel oder Lösungsmittelgemisch eingesetzt werden, wie dies zuvor ganz allgemein und spezieller bei der ersten Variante beschrieben ist. In einer speziellen Ausführung wird Wasser eingesetzt. Beispielsweise kann zur Umsetzung nach der zweiten Variante zunächst das 1-Adamantylammoniumhydrochlorid in einer wässrigen Formaldehydlösung gelöst werden. Dazu kann die wässrige Formaldehydlösung auf eine Temperatur von etwa 30 bis 80 °C erwärmt werden. Anschließend wird dann zur Umsetzung die Ameisensäure zugegeben. Die Reaktionstemperatur liegt nach Zugabe der Ameisensäure vorzugsweise in einem Bereich von 20 bis 200 °C, insbesondere von 40 bis 150 °C.

Der Formaldehydgehalt der wässrigen Lösung liegt vorzugsweise in einem Bereich von etwa 10 bis 40 %, vorzugsweise etwa 20 bis 35 %.

Das Molmengenverhältnis von Formaldehyd zu 1-Adamantylammoniumhydrochlorid (oder 1-Adamantylamin) liegt bevorzugt in einem Bereich von 2 : 1 bis 10 : 1, besonders bevorzugt von 2,01 : 1 bis 5 : 1, insbesondere 2,1 : 1 bis 3 : 1.

Die Isolierung des Produkts kann im einfachsten Fall durch Trennung der organischen Phase von der wässrigen Phase erfolgen. Möglich ist auch die Zugabe eines organischen Lösungsmittels, z. B. eines aromatischen Kohlenwasserstoffs, wie Benzol, Toluol oder Xylol. Durch Zusatz eines organischen Lösungsmittels kann im Bedarfsfall die Phasentrennung verbessert werden. Gewünschtenfalls kann die organische Produktphase einer Aufarbeitung nach üblichen Verfahren unterzogen werden. Dazu zählt z. B. das Waschen mit wässrigen Medien oder die Abtrennung von zugesetztem Lösungsmittel, z. B. durch Verdampfen.

Nach der zweiten Variante wird das 1-Adamantyldimethylamin in Form eines Ammoniumsalzes erhalten. Die Überführung in 1-Adamantyldimethylamin kann mit wässriger Base erfolgen. Geeignete Basen sind z. B. Alkalihydroxide, wie NaOH oder KOH.

In einer dritten Variante des erfindungsgemäßen Verfahrens wird zur Bereitstellung des 1-Adamantyldimethylamins in Schritt A) 1-Adamantylamin oder ein 1-Adamantylammoniumsalz einer katalytischen Methylierung mit Methanol unterzogen.

Bevorzugt wird zur katalytischen Methylierung nach der dritten Variante 1-Adamantylamin eingesetzt.

Zur katalytischen Methylierung nach der dritten Variante wird vorzugsweise wenigstens ein organisches Lösungsmittel eingesetzt. Bevorzugt wird zur Umsetzung nach der dritten Verfahrensvariante Methanol nicht nur als Reaktionspartner, sondern auch als Lösungsmittel eingesetzt. Geeignet sind auch die zuvor genannten nicht wassermischbaren Lösungsmittel, speziell aromatische Kohlenwasserstoffe, wie Benzol, Toluol, Ethylbenzol oder Xylol. In einer speziellen Ausführung wird zur Umsetzung ein Lösungsmittelgemisch eingesetzt, das Xylol und Methanol enthält oder aus Xylol und Methanol besteht.

Die Reaktionstemperatur liegt vorzugsweise in einem Bereich von 20 bis 300 °C, besonders bevorzugt von 50 bis 270 °C, insbesondere von 150 bis 250 °C.

Der Druck liegt vorzugsweise in einem Bereich von 1 bis 300 bar, besonders bevorzugt von 5 bis 250 bar. Temperatur und Druck der Reaktion werden sinnvollerweise so gewählt, dass in der Phase, in der die Reaktion erfolgt, ausreichende Konzentrationen der Reaktanden vorhanden sind (z. B. nicht das 1-Adamantylamin im Wesentlichen flüssig und das Methanol im Wesentlichen gasförmig vorliegt).

In einer speziellen Ausführung erfolgt die Umsetzung nach der dritten Variante in mehreren (z. B. 2, 3, 4, 5, etc.) Reaktoren. Bevorzugt ist eine Kombination aus zwei Reaktoren. Dabei können gleiche oder verschiedene Reaktoren eingesetzt werden. Die Reaktoren können untereinander beliebig verschaltet sein, z. B. parallel oder in Reihe. In einer bevorzugten Ausführung werden zwei in Reihe geschaltete Reaktoren eingesetzt. Werden mehrere Reaktoren eingesetzt, so können diese gleiche oder verschiedene Temperaturen aufweisen. Vorzugsweise ist die Temperatur im n. (n-ten) Reaktor um wenigstens 10 °C, besonders bevorzugt um wenigstens 20 °C, insbesondere um wenigstens 30 °C höher als die Temperatur im (n-1). (n minus ersten) Reaktor. Der Reaktionsdruck kann beim Einsatz mehrerer Reaktoren in den einzelnen Reaktoren gleich oder verschieden sein. In einer speziellen Ausführungsform enthält dabei nur ein Teil der Reaktoren Katalysator. So kann z. B. eine Kombination aus zwei Reaktoren eingesetzt werden, von denen nur einer Katalysator enthält. In dieser Variante kann das Reaktionsgemisch zunächst in einem Reaktor ohne Katalysator vorerwärmt und dann anschließend zur Reaktion in einen Reaktor mit Katalysator überführt werden. Zum Überführen des Reaktionsgemisches kann z. B. ein Gas eingesetzt werden, mit dem das Gemisch vom einen in einen anderen Reaktor gedrückt wird. Zusätzlich kann das Gas auch zur Einstellung des gewünschten Reaktionsdrucks dienen. Ein geeignetes Gas ist Wasserstoff. Wasserstoff nimmt dabei nicht an der eigentlichen Umsetzung teil, kann aber dazu dienen, den eingesetzten Katalysator in einer reduzierten Form zu halten.

Als Katalysatoren für die dritte Variante eignen sich prinzipiell Hydrierkatalysatoren, wie sie zuvor beschrieben sind. Bevorzugt wird ein kupferhaltiger, heterogener Katalysator eingesetzt.

Prinzipiell eignen sich eine Vielzahl von kupferhaltigen Katalysatoren, die zusätzlich wenigstens ein weiteres Element oder I., II., III., IV oder V. Hauptgruppe, oder I., II., IV., V., VI., VII. oder VIII. Nebengruppe sowie der Lanthanide enthalten können (IUPAC: Gruppen 1 bis 15 und die Lanthanide), insbesondere Ca, Mg, Al, La, Ti, Zr, Cr, Mo, W, Mn, Ni, Co, Zn und Kombinationen davon. Eine spezielle Ausführungsform von Katalysatoren, die sich vorteilhaft für einen Einsatz in der dritten Verfahrensvariante eignen, sind Raney-Katalysatoren, speziell Raney-Kupfer sowie kupferhaltige Metalllegierungen in Form eines Raney-Katalysators. Bevorzugt sind Raney-Katalysatoren, deren Metallkomponente zu wenigstens 95 %, insbesondere zu wenigstens 99 %, aus Kupfer besteht. Raney-Kupfer kann in an sich bekannter Weise durch Behandeln von Kupfer-Aluminium-Legierungen mit Alkalihydroxiden hergestellt werden.

Eine weitere spezielle Ausführungsform von Katalysatoren, die sich besonders vorteilhaft für einen Einsatz in der dritten Verfahrensvariante eignen, sind Katalysatoren, die Kupfer in oxidischer Form sowie gegebenenfalls zusätzlich in elementarer Form enthalten.

Geeignet sind z. B. Katalysatoren, die auf einem Träger aus Kieselsäure Nickel und Kupfer neben anderen Metallen als aktive Bestandteile enthalten. Solche Katalysatoren werden z. B. in DE-A 26 28 987 beschrieben. Die aktive Masse dieser Katalysatoren enthält speziell 40 bis 80 Gew.-% Nickel, 10 bis 50 Gew.-% Kupfer und 2 bis 10 Gew.-% Mangan. Die EP-A-0 434 062 beschreibt Hydrierungskatalysatoren, die durch Reduktion eines Precursors aus Oxiden des Kupfers, Aluminiums und wenigstens eines weiteren Metalls, ausgewählt unter Magnesium, Zink, Titan, Zirkon, Zinn, Nickel und Kobalt, erhältlich sind. Geeignet sind auch die in der DE 102 18 849 beschriebenen Hydrierkatalysatoren, die 0,1 bis 10 Gew.-% Chrom, berechnet als Cr₂O₃ 0,1 bis 10 Gew.-% Calcium, berechnet als CaOx und 5 bis 20 Gew.-% Kupfer, berechnet als CuO, abgeschieden auf einem Siliciumdioxid-Trägermaterial und jeweils bezogen auf das Gesamtgewicht des calcinierten Katalysators, enthalten. Aus der DE-A-40 21 230 sind Kupfer-Zirkonoxid-Katalysatoren bekannt, wobei das Verhältnis von Kupferatomen zu Zirkoniumatomen, ausgedrückt als Gewichtsverhältnis, 1 : 9 bis 9 : 1 beträgt. Die DE-A-4 028 295 beschreibt geeignete Kupfer-Mangan-Hydrierkatalysatoren. Die EP-A-552463 beschreibt Katalysatoren, wobei die oxidische Form im Wesentlichen der Zusammensetzung CUₐAl_{b}Zr_{c}Mn_{d}Oₓ entspricht, wobei die folgenden Beziehungen gelten: a > 0; b > 0; c ≥ 0; d > 0; a > b/2; b > a/4; a > c; a > d; und x die zur Wahrung der Elektroneutralität pro Formeleinheit erforderliche Anzahl von Sauerstoffionen bezeichnet. Die EP-A-552463 beschreibt auch Katalysatoren mit einem geringeren Anteil an Aluminiumoxid. Der Katalysator nach dieser Ausführungsform entspricht im Wesentlichen der Zusammensetzung CuaAl_{b}Zr_{c}Mn_{d}Oₓ, wobei die folgenden Beziehungen gelten: a > 0; a/40 ≤ b ≤ a/4; c ≥ 0; d > 0; a > c; 0,5d ≤ a ≤ 0,95d und x, die zur Wahrung der Elektroneutralität pro Formeleinheit erforderliche Anzahl von Sauerstoffionen bezeichnet. Die WO 2006/005505 beschreibt Katalysatorformkörper, die sich für den Einsatz in dem erfindungsgemäßen Verfahren besonders eignen. In einer bevorzugten Ausführung umfasst das oxidische Katalysatormaterial
(a) Kupferoxid mit einem Anteil im Bereich von 50 ≤ x ≤ 80 Gew.-%, bevorzugt 55 ≤ x ≤ 75 Gew.%,
(b) Aluminiumoxid mit einem Anteil im Bereich von 15 ≤ y ≤ 35 Gew.-%, bevorzugt 20 ≤ y ≤ 30 Gew.-%, und
(c) mindestens eines der Oxide des Lanthans, Wolframs, Molybdäns, Titans oder Zirkoniums, bevorzugt des Lanthans und/oder Wolframs, mit einem Anteil im Bereich von 2 ≤ z ≤ 20 Gew.-%, bevorzugt 3 ≤ z ≤ 15 Gew.-%,
jeweils bezogen auf das Gesamtgewicht des oxidischen Materials nach Calcinierung, wobei gilt: 80 ≤ x + y + z ≤ 100, besonders 95 ≤ x + y + z ≤ 100.

Bevorzugte Katalysatoren für die dritte Verfahrensvariante umfassen die folgenden Metalle in oxidischer Form, reduzierter Form (elementarer Form) oder einer Kombination davon. Metalle, die in mehr als einer Oxidationsstufe stabil sind, können vollständig in einer der Oxidationsstufen oder in verschiedenen Oxidationsstufen eingesetzt werden:
Cu
Cu, Ti
Cu, Zr
Cu, Mn
Cu, Al
Cu, Ni, Mn
Cu, Al, wenigstens ein weiteres Metall, ausgewählt unter La, W, Mo, Mn, Zn, Ti, Zr, Sn, Ni, Co
Cu, Zn, Zr
Cu, Cr, Ca
Cu, Cr, C
Cu, Al, Mn, gegebenenfalls Zr.

Als inertes Trägermaterial für die erfindungsgemäßen Katalysatoren können praktisch alle Trägermaterialien des Stands der Technik, wie sie vorteilhaft bei der Herstellung von geträgerten Katalysatoren Verwendung finden, beispielsweise SiO₂ (Quarz), Porzellan, Magnesiumoxid, Zinndioxid, Siliciumcarbid, TiO₂ (Rutil, Anatas), Al₂O₃ (Tonerde), Aluminiumsilikat, Steatit (Magnesiumsilikat), Zirkoniumsilikat, Cersilikat oder Mischungen dieser Trägermaterialien, eingesetzt werden. Bevorzugte Trägermaterialien sind Aluminiumoxid und Siliciumdioxid.

Die Katalysatoren können als Formkörper eingesetzt werden, z. B. in Form von Kugeln, Ringen, Zylindern, Würfeln, Quadern oder anderen geometrischen Körpern. Ungeträgerte Katalysatoren können nach üblichen Verfahren geformt werden, z. B. durch Extrudieren, Tablettieren, etc. Die Form geträgerter Katalysatoren wird durch die Form des Trägers bestimmt. Alternativ dazu kann der Träger vor oder nach dem Aufbringen der katalytisch aktiven Komponente(n) einem Formungsverfahren unterzogen werden. Die Katalysatoren können z. B. in Form von gepressten Zylindern, Tabletten, Pastillen, Wagenrädern, Ringen, Sternen oder Strangpresslingen, wie Vollsträngen, polylobären Strängen, Hohlsträngen und Wabenkörpern oder anderen geometrischen Körpern, eingesetzt werden.

Bevorzugt erfolgt in Schritt A) die Umsetzung des 1-Adamantyldimethylamins mit Dimethylsulfat bei einer erhöhten Temperatur, d. h. bei einer Temperatur, die oberhalb der Umgebungstemperatur (20 °C) liegt. Vorzugsweise beträgt die Temperatur in Schritt A) wenigstens 40 °C, besonders bevorzugt wenigstens 80 °C. Vorzugsweise erfolgt die Umsetzung in Schritt A) bei einer Temperatur im Bereich von oberhalb 100 bis 220 °C, besonders bevorzugt von 120 bis 200 °C.

In einer bevorzugten Ausführung wird in Schritt A) zunächst das 1-Adamantyldimethylamin mit dem Dimethylsulfat, bei einer Temperatur von höchstens 30 °C in Kontakt gebracht und anschließend das resultierende Gemisch zur weiteren Umsetzung auf eine Temperatur von wenigstens 40 °C erwärmt. Bevorzugt erfolgt das Inkontaktbringen des 1-Adamantyldimethylamins mit Dimethylsulfat bei einer Temperatur von höchstens 20 °C, insbesondere bei einer Temperatur von höchstens 10 °C. Bevorzugt erfolgt das Inkontaktbringen des 1-Adamantyldimethylamins mit dem Dimethylsulfat portionsweise. Dazu kann das Amin oder Dimethylsulfat vorgelegt und die jeweils andere Komponente portionsweise zugegeben werden. Bevorzugt wird das Gemisch zur weiteren Umsetzung auf eine Temperatur von wenigstens 80 °C, besonders bevorzugt 100 bis 220 °C, insbesondere von 120 bis 200 °C, erwärmt.

Die Umsetzung des 1-Adamantyldimethylamins mit dem Dimethylsulfat in Schritt A) erfolgt vorzugsweise in Gegenwart eines unter den Reaktionsbedingungen inerten Lösungsmittels. Geeignete Lösungsmittel sind z. B. Wasser, wassermischbare Lösungsmittel und Mischungen davon. Bevorzugte wassermischbare Lösungsmittel sind Alkohole, wie Methanol, Ethanol, n-Propanol, Isopropanol, n-Butanol und Mischungen davon. Bevorzugt wird als Lösungsmittel Wasser oder ein Lösungsmittelgemisch eingesetzt, das mindestens 30 Vol-%, bevorzugt mindestens 50 Vol-%, insbesondere mindestens 80 Vol-% Wasser umfasst. In einer speziellen Ausführung wird in Schritt A) Wasser als Lösungsmittel eingesetzt.

Bevorzugt werden das 1-Adamantyldimethylamin und das Dimethylsulfat beide in flüssiger Form eingesetzt. Das Dimethylsulfat wird vorzugsweise in reiner Form eingesetzt. Das 1-Adamantyldimethylamin wird vorzugsweise in Kombination mit Wasser oder einem Gemisch von Wasser mit wenigstens einem wassermischbaren Lösungsmittel eingesetzt. Vorzugsweise wird das 1-Adamantyldimethylamin, gegebenenfalls in Kombination mit einem Lösungsmittel, vorgelegt und das Dimethylsulfat zugegeben.

Die Umsetzung in Schritt A) erfolgt vorzugsweise bei Umgebungsdruck oder unter erhöhtem Druck. In einer speziellen Ausführung erfolgt die Reaktion unter dem Eigendruck der Reaktionsmischung bei den Reaktionsbedingungen. Bevorzugt beträgt der Druck bei der Umsetzung in Schritt A) im Allgemeinen mindestens 1,1 bar, besonders bevorzugt mindestens 2 bar, insbesondere mindestens 5 bar. Gewünschtenfalls kann der Druck bei der Umsetzung in Schritt A) bis 300 bar, bevorzugt bis 100 bar, betragen. Geeignete druckfeste Reaktoren sind dem Fachmann bekannt und werden z. B. in Ullmann's Enzyklopädie der technischen Chemie, Band 1, 3. Auflage, 1951, S. 769 ff beschrieben. Im Allgemeinen wird für die Durchführung des erfindungsgemäßen Verfahrens unter erhöhtem Druck ein Druckbehälter verwendet, der gewünschtenfalls mit einer Rührvorrichtung und/oder einer Innenauskleidung versehen sein kann.

Das Molmengenverhältnis von 1-Adamantyldimethylamin zu Dimethylsulfat beträgt vorzugsweise wenigstens 2:1. Besonders bevorzugt liegt das Molmengenverhältnis von 1-Adamantyldimethylamin zu Dimethylsulfat in einem Bereich von 2 : 1 bis 20 : 1, insbesondere 2,05 : 1 bis 10 : 1, speziell 2,1 : 1 bis 5 : 1.

Gewünschtenfalls kann die Umsetzung in Schritt A) in Gegenwart wenigstens eines Inertgases erfolgen. Geeignete Inertgase sind beispielsweise Stickstoff, Helium und Argon.

Die Umsetzung in Schritt A) kann kontinuierlich oder diskontinuierlich erfolgen.

Es ist in der Regel möglich, das in Schritt A) erhaltene Reaktionsgemisch ohne vorherige Isolierung des 1-Adamantyltrimethylammoniumsulfats zum Ionenaustausch in Schritt B) einzusetzen.

Das in Schritt A) erhaltene Reaktionsgemisch kann vor der Umsetzung in Schritt B) wenigstens einem Aufarbeitungsschritt unterzogen werden. Dazu zählt z. B. die teilweise oder vollständige Entfernung von Lösungsmittel. Dazu zählt weiterhin die Abtrennung von nicht umgesetztem 1-Adamantyldimethylamin oder von unerwünschten Nebenprodukten, wie 1-Adamantyltrimethylammoniummethylsulfat. In der Regel gelingt es in Schritt A), die Bildung von 1-Adamantyltrimethylammoniummethylsulfat so weit zu vermeiden, dass das erhaltene Reaktionsgemisch ohne Abtrennung wenigstens eines Teils dieser Verbindung zum Ionenaustausch in Schritt B) eingesetzt werden kann.

Die Aufarbeitung des in Schritt A) erhaltenen Reaktionsgemischs kann nach üblichen, dem Fachmann bekannten Verfahren erfolgen. Bevorzugt ist eine Aufarbeitung zur Aufkonzentrierung des 1-Adamantyltrimethylammoniummethylsulfat, z. B. durch Verdampfen von flüchtigen Komponenten.

In einer Ausführung des erfindungsgemäßen Verfahrens wird das in Schritt A) erhaltene Reaktionsgemisch einer teilweisen oder vollständigen Entfernung des Lösungsmittels unterzogen. Dies gilt speziell, wenn der Ionenaustausch in Schritt B) in einem anderen Lösungsmittel erfolgen soll als die Alkylierung in Schritt A). Dies gilt auch, wenn der Ionenaustausch in Schritt B) in einer nicht zu großen Lösungsmittelmenge erfolgen soll. Das Lösungsmittel kann beispielsweise durch Verdampfen, vorzugsweise unter verringertem Druck, teilweise oder vollständig entfernt werden. Da die erhaltenen 1-Adamantyltrimethylammoniumsalze nicht flüchtig sind, ist der eingesetzte Druckbereich in der Regel nicht kritisch. Sofern eine möglichst vollständige Entfernung des Lösungsmittels gewünscht ist, kann beispielsweise ein Feinvakuum von 10¹ bis 10⁻¹ Pa oder ein Hochvakuum von 10⁻¹ bis 10⁻⁵ Pa eingesetzt werden. Zur Vakuumerzeugung können übliche Vakuumpumpen, wie Flüssigkeitsstrahlvakuumpumpen, Dreh- und Sperrschiebervakuumpumpen, Membranvakuumpumpen, Diffusionspumpen etc. eingesetzt werden. Das Entfernen des Lösungsmittels kann gewünschtenfalls bei einer erhöhten Temperatur erfolgen. Bevorzugt erfolgt das Entfernen des Lösungsmittels bei einer Temperatur von bis zu 150 °C, besonders bevorzugt bis zu 100 °C.

In einer speziellen Ausführung erfolgt die Umsetzung in Schritt A) in Wasser als Lösungsmittel und wird das in Schritt A) erhaltene Reaktionsgemisch einer destillativen Aufarbeitung zur Entfernung eines Teils des Wassers und, falls vorhanden, von nicht umgesetztem 1-Adamantyldimethylamin unterzogen. Nach dieser Ausführung wird in Schritt A) eine wässrige Lösung des 1-Adamantyltrimethylammoniumsulfats erhalten.

Bevorzugt wird in Schritt A) ein Methylierungsprodukt erhalten, das wenigstens 95 Gew.-%, besonders bevorzugt wenigstens 99 Gew.-%, insbesondere wenigstens 99,9 Gew.-%, 1-Adamantyltrimethylammoniumsulfat, bezogen auf das Gesamtgewicht aus 1-Adamantyltrimethylammoniumsulfat und 1-Adamantyltrimethylammoniummethylsulfat enthält.

### Schritt B)

Erfindungsgemäß wird das in Schritt A) erhaltene 1-Adamantyltrimethylammoniumsulfat einem Ionentausch unterzogen. Dabei werden die Sulfationen und, falls das zum Austausch eingesetzte Ausgangsmaterial noch Methylsulfationen enthält, die Methylsulfationen gegen Hydroxidionen ausgetauscht.

Zum Ionenaustausch eignen sich prinzipiell die dem Fachmann bekannten basischen Ionenaustauscher, die wenigstens eine an eine Festphase immobilisierte Base aufweisen. Die Festphase dieser basischen Ionenaustauscher umfasst beispielweise eine Polymermatrix. Dazu zählen z. B. Polystyrolmatrices, die neben Styrol wenigstens ein vernetzendes Monomer, z. B. Divinylbenzol, sowie gegebenenfalls weitere Comonomere einpolymerisiert enthalten. Geeignet sind weiterhin Polyacrylmatrices, die durch Polymerisation wenigstens eines (Meth)acrylats, wenigstens eines vernetzenden Monomers sowie gegebenenfalls weiterer Comonomere erhalten werden. Geeignete Polymermatrices sind auch Phenol-Formaldehyd-Harze und Polyalkylaminharze, die beispielsweise durch Kondensation von Polyaminen mit Epichlorhydrin erhalten werden.

Die an die Festphase direkt oder über eine Spacergruppe gebundenen so genannten Ankergruppen (deren locker gebundene Gegenionen gegen gleichsinnig geladene Ionen ausgetauscht werden können) sind vorzugsweise ausgewählt unter stickstoffhaltigen Gruppen, vorzugsweise tertiären und quartären Aminogruppen.

Geeignete funktionelle Gruppen sind z. B.:
- CH₂N⁺(CH₃)₃ OH⁻ z. B. Duolite A 101, Ambersep 900 OH
- CH₂N⁺(CH3)₂CH₂CH₂OH OH⁻ z. B. Duolite A 102, Amberlite IRA 410
- CH₂N⁺(C₃H₇)₃ OH⁻ z. B. lonac SR 7

Für das erfindungsgemäße Verfahren eignen sich sowohl stark als auch schwach basische Ionenaustauscher. Bevorzugt sind stark basische Ionenaustauscher. Stark basische (An)ionenaustauscher sind Anionenaustauscher, die in der unbeladenen Form stark basische funktionelle Gruppen besitzen, beispielsweise quartäre Ammoniumgruppen mit Hydroxidionen als Gegenionen. Im Gegensatz zu schwach basischen Anionenaustauschern können sie in der Regel ohne vorherige Protonierung auch in neutraler oder basischer Lösung eingesetzt werden. Stark basische Anionenaustauscher können durch Behandeln mit wässrigen Lösungen starker Basen, wie NaOH oder KOH, regeneriert werden. Schwach basische Anionenaustauscher sind Anionenaustauscher, die schwach basische funktionelle Gruppen besitzen. Dazu zählen z. B. primäre, sekundäre oder tertiäre Aminogruppen. Sie können durch die zuvor genannten starken Basen, aber auch durch schwache Basen, wie Alkalicarbonate oder Ammoniaklösung, regeneriert werden. Unter den schwach basischen Ionenaustauschern sind solche, die tertiäre Aminogruppen aufweisen, bevorzugt.

Bevorzugt für den Einsatz in dem erfindungsgemäßen Verfahren sind stark basische Ionenaustauscher.

Für das erfindungsgemäße Verfahren geeignete kommerziell erhältliche Ionenaustauscher sind z. B. Amberlyst® A27 (quaternäre Ammoniumgruppen, stark basisch) und Ambersep® 900 OH (stark basisch). Weitere geeignete Anionenaustauscher sind beispielsweise die Amberlite-, Duolite-und Purolite-Harze der Fa. Rohm & Haas, wie Amberlite IRA-402, IRA-458, IRA-900. Geeignet sind weiterhin IMAC HP 555, Amberjet 4200 und Amberjet 4400. Weitere geeignete Anionenaustauscher sind beispielsweise die Lewatit-Harze der BAYER AG, wie Lewatit M 500, MP500 etc. Weitere geeignete Anionenaustauscher sind beispielsweise die Dowex-Marken der Fa. Dow, wie DOWEX SBR-P, SAR, MARATHON MSA, 22, etc.

Zum Ionenaustausch werden die Ionenaustauscher, falls erforderlich, zunächst mit OH-Ionen beladen und anschließend mit einer Lösung in Kontakt gebracht, die das in Schritt A) erhaltene 1-Adamantyltrimethylammoniumsulfat enthält. Als Lösungsmittel wird vorzugsweise Wasser eingesetzt.

Bevorzugt wird zum Ionenaustausch in Schritt B) eine wässrige Lösung eingesetzt, die 1-Adamantyltrimethylammoniumsulfat in einer Konzentration von 2 bis 20 Gew.-%, besonders bevorzugt 5 bis 15 Gew.-%, enthält.

Die Behandlung mit dem Anionenaustauscher erfolgt in üblicher Weise, beispielsweise indem man die 1-Adamantyltrimethylammoniumsulfat enthaltende wässrige Lösung im Gefäß mit dem Ionenaustauscher in Kontakt bringt oder vorzugsweise über ein Bett des Ionenaustauschers, speziell eine mit dem Ionenaustauscher beschickte Säule, leitet.

Der Ionenaustausch in Schritt B) kann kontinuierlich oder diskontinuierlich durchgeführt werden. Bevorzugt ist die kontinuierliche Betriebsweise. Dazu kann eine Kombination aus mehreren (z. B. 2, 3, 4, 5, etc.) Ionenaustauschersäulen eingesetzt werden, wobei zeitgleich ein Teil der Säulen zum Ionenaustausch verwendet wird und ein anderer Teil der Regenerierung unterzogen wird.

In einer speziellen Ausführung des erfindungsgemäßen Verfahrens wird in Schritt B) eine wässrige 1-Adamantyltrimethylammoniumhydroxid-Lösung erhalten, die zur Aufkonzentrierung einer teilweisen Abtrennung von Wasser unterzogen werden kann.

In der Regel weist die nach dem Anionenaustausch an dem mit OH-Ionen beladenen Ionenaustauscher erhaltene wässrige 1-Adamantyltrimethylammoniumhydroxid-Lösung einen 1-Adamantyltrimethylammoniumhydroxid-Gehalt von wenigstens 1 Gew.-%, bevorzugt wenigstens 3 Gew.-%, insbesondere wenigstens 5 Gew.-%, bezogen auf das Gesamtgewicht der Lösung, auf.

Die teilweise Abtrennung von Wasser kann nach üblichen, dem Fachmann bekannten Verfahren erfolgen. Dazu eignen sich übliche Verdampfer, die im einfachsten Fall ein Behältnis mit beheizbaren Wärmetauscherflächen umfassen. Vorzugsweise wird ein Dünnschichtverdampfer eingesetzt, beispielsweise ein Fallfilmverdampfer. Geeignet sind weiterhin Verdampfer mit beweglichen Einbauten, in denen z. B. Wischblätter einen dünnen Flüssigkeitsfilm an der Innenwand des Verdampfers erzeugen. Dazu zählen Dünnschichtverdampfer der Typen "LUWA"® oder "SAMBAY"®. Die Verdampfung erfolgt vorzugsweise bei einer Temperatur im Bereich von 0 bis 150 °C, besonders bevorzugt 10 bis 120 °C, insbesondere 20 bis 100 °C. Die Verdampfung erfolgt vorzugsweise bei einem Druck im Bereich von 0,01 mbar bis 1013 mbar, besonders bevorzugt von 0,1 mbar bis 500 mbar, insbesondere 1 bis 250 mbar.

Die nach dem erfindungsgemäßen Verfahren (gegebenenfalls nach einer teilweisen Abtrennung von Wasser) erhaltene 1-Adamantyltrimethylammoniumhydroxid-Lösung weist vorzugsweise einen 1-Adamantyltrimethylammoniumhydroxid-Gehalt von wenigstens 10 Gew.-%, besonders bevorzugt wenigstens 15 Gew.-%, insbesondere wenigstens 18 Gew.-%, bezogen auf das Gesamtgewicht der Lösung, auf.

### Beispiele

### Beispiel 1:

### Freisetzung von 1-Adamantylamin aus 1-Adamantylammoniumhydrochlorid mit Natronlauge

In einem für das Arbeiten unter Inertgas ausgerüsteten 10 m³ Rührkessel (80%ige Befüllung) werden 1380 kg 1-Adamantylammoniumhydrochlorid unter Stickstoffatmosphäre vorgelegt und 5170 I Xylol zugegeben. Bei Atmosphärendruck werden unter Rühren 1840 I 20%ige Natronlauge innerhalb von 30 Minuten zudosiert und das Reaktionsgemisch anschließend zwei Stunden nachgerührt, wobei sich das Hydrochlorid löst und 1-Adamantylamin freigesetzt wird. Nach Phasentrennung wird die wässrige Phase abgelassen und die organische Phase mit 300 l 1%iger Natronlauge gerührt. Die wässrige Phase wird wiederum abgelassen und die organische Phase zur Entfernung von Wasser mit 300 I 50%iger Natronlauge ausgerührt. Anschließend wird die wässrige Phase wiederum abgelassen. Es resultieren 4384 I einer ca. 32%igen xylolischen Lösung von 1-Adamantylamin mit einem Restwassergehalt von etwa 0,13 %. Die vereinigten wässrigen Phasen werden einmal mit 1720 I Xylol gerührt, um gelöstes 1-Adamantylamin zu entfernen. Die dabei erhaltene organische Phase wird bei der Freisetzung der nächsten Charge 1-Adamantylamin als Lösungsmittel eingesetzt.

Die Ausbeute an freigesetztem Adamantylamin beträgt 99,8 %.

### Beispiel 2:

### Herstellung von 1-Adamantyldimethylamin durch reduktive Aminierung von Formaldehyd mit 1-Adamantylamin und Wasserstoff in Gegenwart von Raney-Nickel

In einem für das Arbeiten unter Inertgas ausgerüsteten 10 m³ Rührkessel (80%ige Befüllung) werden unter Stickstoff 5501 kg 25%ige xylolische 1-Adamantylaminlösung aus Beispiel 1 vorgelegt. Unter Rühren werden 138 kg Raney-Nickel als Katalysator und 55 kg einer 30%igen wässrigen NaH₂PO₄-Lösung zugegeben. Der Reaktor wird mit Wasserstoff begast, bis ein Innendruck von 5 bar erreicht ist und danach auf 100 °C aufgeheizt. Durch weitere Zugabe von Wasserstoff wird ein Reaktorinnendruck von 50 bar eingestellt. Anschließend werden unter Rühren innerhalb von drei Stunden 1905 kg einer 30%igen Formaldehydlösung zudosiert und anschließend noch zwei Stunden bei 100 °C nachgerührt. Nach Abkühlen des Reaktionsgemischs und Entspannen des Reaktors wird der flüssige Reaktorinhalt entleert, der Austrag filtriert, wobei ein zweiphasiges Filtrat erhalten wird. Dieses Filtrat wird in einem Kessel unter Rühren mit 73 kg einer 25%igen wässrigen Natronlauge versetzt. Nach 15 Minuten Rühren lässt man die Phasen separieren und unterzieht die organische Phase einer Destillation zur Abtrennung des Xylols. Das 1-Adamantyldimethylamin wird dabei als Sumpfprodukt erhalten. Ausbeute: >99 %

### Beispiel 3:

Herstellung von 1-Adamantyldimethylamin durch reduktive Aminierung von Formaldehyd mit 1-Adamantylamin in Gegenwart von Ameisensäure (Eschweiler-Clark):
In einem für das Arbeiten unter Inertgas ausgerüsteten 2,5 m³ Rührreaktor werden unter Stickstoff 570 kg 1-Adamantylammoniumhydrochlorid vorgelegt und unter Rühren 901 kg einer 30%igen Formaldehydlösung bei Raumtemperatur zugegeben. Anschließend wird das Reaktionsgemisch auf 60 °C aufgeheizt und eine Stunde gerührt, um das Hydrochlorid zu lösen. Danach werden 422 kg Ameisensäure bei 60 °C zugegeben. Der Reaktor wird langsam auf 90 °C aufgeheizt und 115 bis 125 h bei dieser Temperatur gerührt, wobei die Reaktion gaschromatographisch kontrolliert wird. Nach beendeter Umsetzung lässt man den Reaktor auf 45 °C abkühlen, gibt 732 kg einer 50%igen Natronlauge mit einer Dosierrate von ca. 100 kg/Stunde zu, wobei die Innentemperatur 50 °C nicht überschreitet. Danach lässt man den Reaktor auf 25 °C abkühlen und die Phasen trennen. Die organische Phase wird isoliert und zur Abtrennung von verbliebenem Wasser einer Destillation bei 70 mbar und 80 °C Sumpftemperatur unterzogen. Im Verlauf der Destillation wird der Druck weiter auf 5 mbar verringert und anschließend der Sumpf auf 100 °C aufgeheizt. Nach beendeter Destillation wird das 1-Adamantyldimethylamin als Kopfprodukt erhalten. Ausbeute: 97 bis 98%, Reinheit nach Gaschromatographie: 99,5 bis 99,6 Flächen-%

### Beispiel 4:

### Herstellung von 1-Adamantyldimethylamin durch katalytische Methylierung von 1-Adamantylamin mit Methanol

### 4.1 Batch Fahrweise:

Die Reaktion erfolgt in zwei miteinander über dünne Rohrleitungen verbundenen 270 ml Autoklaven. In dem zweiten Autoklaven werden 10 g eines CuO-Katalysators auf Al₂O₃ vorgelegt und der Katalysator zwei Stunden bei 200 °C und 200 bar Wasserstoff aktiviert (der Katalysator wird 3x ohne Aktivitätsverlust wiederverwendet). In den ersten Autoklaven wird eine Lösung aus 15 % 1-Adamantylamin, 35 % Xylol und 50 % Methanol eingefüllt und anschließend der erste Autoklav auf 90 °C und der zweite Autoklav auf 220 °C erwärmt. Nach 10 Minuten wird das Reaktionsgemisch mit Hilfe von Wasserstoff aus dem ersten in den zweiten Autoklaven überführt. In dem zweiten Autoklaven wird die Reaktion unter Rühren vier Stunden bei 200 °C und 100 bar durchgeführt. Es wird ein quantitativer Umsatz von 1-Adamantylamin zu 1-Adamantyldimethylamin erzielt. Ausbeute: > 99%

Zur Isolierung des 1-Adamantyldimethylamins wird das Reaktionsprodukt einer destillativen Entfernung des Methanols, des Xylols und des bei der Reaktion freigesetzten Wassers unterzogen.

### 4.2 Kontinuierliche Fahrweise (Laboranlage):

In einen Rohrreaktor (18 x 3 x 770 mm) werden 51,4 g CuO-Katalysator auf Al₂O₃ (Tabletten) eingefüllt (Katalysatorschütthöhe: ca. 500 mm). Der Rohrreaktor wird kontinuierlich mit einem Gemisch der Einsatzstoffe durchströmt (Sumpffahrweise, 94,0 g/h), die Zusammensetzung des Zulaufs beträgt 15 % Adamantylamin, 35 % Xylol und 50 % Methanol. Bei einer Belastung von 0,25 kg Adamantylamin / l*h, werden bei 80 bar und 230 °C >99 % 1-Adamantyldimethylamin erhalten.

### 4.3 Kontinuierliche Fahrweise (Technikum):

In einen Rohrreaktor (4.85I) werden 4394.0 g CuO-Katalysator auf Al₂O₃ (Tabletten) eingefüllt. Der Rohrreaktor wird kontinuierlich mit einem Gemisch der Einsatzstoffe durchströmt [Sumpffahrweise, 9365.0 g/h], die Zusammensetzung des Zulaufs beträgt 13% Adamantylamin, 37% Xylol und 50% Methanol. Bei einer Belastung von 0,25 kg Adamantylamin / l*h, werden bei 80 bar und 195°C >99% 1-Adamantyldimethylamin erhalten.

### Beispiel 5:

### Quarternisierung des 1-Adamantyldimethylamins mit Dimethylsulfat

In einem 2,5 m³-Rührbehälter werden 2,5 t Wasser und 533 kg 1-Adamantyldimethylamin vorgelegt. Anschließend werden 191 kg Dimethylsulfat zudosiert und das resultierende Reaktionsgemisch 2 Stunden bei 30 bis 50 °C umgesetzt. Anschließend wird der Kessel auf 140 °C aufgeheizt, wobei ein Innendruck von 4 bar erreicht wird und bei dieser Temperatur weitere 16 Stunden umgesetzt. Das Quarternisierungsprodukt wird über Destillation einer Abtrennung von Wasser unterzogen, wobei eine 30 bis 33 gew.-%ige Lösung von 1-Adamantyltrimethylammoniumsulfat erhalten wird. Umsatz: 99 %

### Beispiel 6:

### Herstellung von 1-Adamantyltrimethylammoniumsulfat durch Ionenaustausch

1-Adamantyltrimethylammoniumsulfat wird als 30%ige Lösung eingesetzt und in einem statischen Mischer mit recycliertem Waschwasser vermischt, so dass eine 10%ige Lösung entsteht. Diese Lösung wird bei Umgebungstemperatur von unten in eine Ionentauschersäule gefahren. Die Menge beträgt ca. 1,3 Bettvolumen. Hierbei werden die Sulfat- und (soweit vorhanden) Methylsulfationen gegen Hydroxidionen ausgetauscht. Danach wird die Säule mit vollentsalztem Wasser und recycliertem Waschwasser (insgesamt ca. 3 Bettvolumina) produktfrei gespült. Ein Teil des Ablaufs des lonentauschers wird als Wertprodukt isoliert, der andere Teil wird als Waschwasser aufgefangen und im nachfolgenden Zyklus als Wasch- oder Verdünnungswasser eingesetzt. Anschließend wird die lonentauschersäule mit 7 gew.-%iger Natronlauge (3 Bettvolumina) regeneriert und mit 4,5 Bettvolumina vollentsalztem Wasser und recycliertem Waschwasser laugenfrei gewaschen. Das Wertprodukt wird durch Abdestillieren von Wasser bei einem Absolutdruck von 130 mbar und einer Temperatur von ca. 50 °C auf 20 % aufkonzentriert.

## Patentansprüche

1. Verfahren zur Herstellung von 1-Adamantyltrimethylammoniumhydroxid, bei dem man
A) 1-Adamantyldimethylamin bereitstellt und einer Umsetzung mit Dimethylsulfat unterzieht, wobei im Wesentlichen beide Methylgruppen des Dimethylsulfats abreagieren und im Wesentlichen 1-Adamantyltrimethylammoniumsulfat erhalten wird, und
B) das in Schritt A) erhaltene 1-Adamantyltrimethylammoniumsulfat einem Anionenaustausch an einem mit OH-Ionen beladenen Ionenaustauscher unterzieht.

2. Verfahren nach Anspruch 1, bei dem man zur Bereitstellung des 1-Adamantyldimethylamins in Schritt A) 1-Adamantylamin oder ein 1-Adamantylammoniumsalz einer Dimethylierung unterzieht.

3. Verfahren nach Anspruch 2, bei dem man zur Bereitstellung des 1-Adamantyldimethylamins von 1-Adamantylammoniumhydrochlorid ausgeht.

4. Verfahren nach Anspruch 3, wobei das 1-Adamantylammoniumhydrochlorid zunächst durch Umsetzung mit wässriger Base in 1-Adamantylamin überführt wird.

5. Verfahren nach einem der Ansprüche 1 bis 4, bei dem man zur Bereitstellung des 1-Adamantyldimethylamins in Schritt A) Formaldehyd einer reduktiven Aminierung mit 1-Adamantylamin oder einem 1-Adamantylammoniumsalz und mit Wasserstoff in Gegenwart eines Hydrierkatalysators unterzieht.

6. Verfahren nach Anspruch 5, wobei die Umsetzung in einem System erfolgt, das zwei flüssige Phasen aufweist.

7. Verfahren nach einem der Ansprüche 5 oder 6, bei dem man
a) 1-Adamantylammoniumhydrochlorid durch Behandeln mit einer wässrigen Lösung einer Base in das 1-Adamantylamin überführt,
b) das 1-Adamantylamin einer reduktiven Aminierung mit Formaldehyd und Wasserstoff in Gegenwart eines Hydrierkatalysators zu 1-Adamantyldimethylamin unterzieht,
c) das 1-Adamantyldimethylamin einer Umsetzung mit Dimethylsulfat unterzieht, wobei im Wesentlichen beide Methylgruppen des Dimethylsulfats abreagieren und im Wesentlichen 1-Adamantyltrimethylammoniumsulfat erhalten wird, und
d) das 1-Adamantyltrimethylammoniumsulfat einem Anionenaustausch an einem mit OH-Ionen beladenen Ionenaustauscher unterzieht.

8. Verfahren nach einem der Ansprüche 1 bis 4, bei dem man zur Bereitstellung des 1-Adamantyldimethylamins in Schritt A) Formaldehyd einer reduktiven Aminierung mit 1-Adamantylamin oder einem 1-Adamantylammoniumsalz in Gegenwart von Ameisensäure unterzieht.

9. Verfahren nach einem der Ansprüche 1 bis 4, bei dem man zur Bereitstellung des 1-Adamantyldimethylamins in Schritt A) 1-Adamantylamin oder ein 1-Adamantylammoniumsalz einer katalytischen Methylierung mit Methanol unterzieht.

10. Verfahren nach einem der vorhergehenden Ansprüche, wobei die Umsetzung mit Dimethylsulfat in Schritt A) bei einer Temperatur von wenigstens 40 °C, besonders bevorzugt von wenigstens 80 °C, erfolgt.

11. Verfahren nach einem der vorhergehenden Ansprüche, wobei in Schritt A) das Molmengenverhältnis von 1-Adamantyldimethylamin zu Dimethylsulfat wenigstens 2 : 1 beträgt.

12. Verfahren nach einem der vorhergehenden Ansprüche, wobei in Schritt A) ein Methylierungsprodukt erhalten wird, das wenigstens 95 Gew.-%, besonders bevorzugt wenigstens 99 Gew.-%, insbesondere wenigstens 99,9 Gew.-%, 1-Adamantyltrimethylammoniumsulfat, bezogen auf das Gesamtgewicht aus 1-Adamantyttrimethylammoniumsulfat und 1-Adamantyltrimethylammoniummethylsulfat enthält.

13. Verfahren nach einem der vorhergehenden Ansprüche, wobei in Schritt B) ein stark basischer Anionenaustauscher eingesetzt wird.

14. Verfahren nach einem der vorhergehenden Ansprüche, wobei in Schritt B) eine wässrige 1-Adamantyltrimethylammoniumhydroxid-Lösung erhalten wird, die gegebenenfalls einer teilweisen Abtrennung von Wasser unterzogen wird.

15. Verfahren nach Anspruch 14, wobei die, gegebenenfalls nach einer teilweisen Abtrennung von Wasser, erhaltene 1-Adamantyltrimethylammoniumhydroxid-Lösung einen 1-Adamantyltrimethylammoniumhydroxid-Gehalt von wenigstens 10 Gew.-%, bevorzugt wenigstens 15 Gew.-%, insbesondere wenigstens 18 Gew.-%, bezogen auf das Gesamtgewicht der Lösung, aufweist.

## Claims

1. A process for preparing 1-adamantyltrimethylammonium hydroxide, by
A) providing 1-adamantyldimethylamine and subjecting it to a reaction with dimethyl sulfate in which essentially both methyl groups of the dimethyl sulfate are consumed by reaction, to give, essentially, 1-adamantyltrimethylammonium sulfate, and
B) subjecting the 1-adamantyltrimethylammonium sulfate obtained in step A) to anion exchange on an ion exchanger loaded with OH ions.

2. The process according to claim 1, wherein the 1-adamantyldimethylamine is provided in step A) by subjecting 1-adamantylamine or a 1-adamantylammonium salt to a dimethylation.

3. The process according to claim 2, wherein the 1-adamantyldimethylamine is provided by starting from 1-adamantylammonium hydrochloride.

4. The process according to claim 3, the 1-adamantylammonium hydrochloride being first converted by reaction with aqueous base into 1-adamantylamine.

5. The process according to any of claims 1 to 4, wherein the 1-adamantyldimethylamine is provided in step A) by subjecting formaldehyde to reductive amination with 1-adamantylamine or a 1-adamantylammonium salt and with hydrogen in the presence of a hydrogenation catalyst.

6. The process according to claim 5, the reaction taking place in a system which has two liquid phases.

7. The process according to either of claims 5 and 6, wherein
a) 1-adamantylammonium hydrochloride is converted by treatment with an aqueous solution of a base into 1-adamantylamine,
b) the 1-adamantylamine is subjected to reductive amination with formaldehyde and hydrogen in the presence of a hydrogenation catalyst, to give 1-adamantyldimethylamine,
c) the 1-adamantyldimethylamine is subjected to reaction with dimethyl sulfate, essentially both methyl groups of the dimethyl sulfate being consumed by reaction, to give, essentially, 1-adamantyltrimethylammonium sulfate, and
d) the 1-adamantyltrimethylammonium sulfate is subjected to anion exchange on an ion exchanger loaded with OH ions.

8. The process according to any of claims 1 to 4, wherein the 1-adamantyldimethylamine is provided in step A) by subjecting formaldehyde to reductive amination with 1-adamantylamine or a 1-adamantylammonium salt in the presence of formic acid.

9. The process according to any of claims 1 to 4, wherein the 1-adamantyldimethylamine is provided in step A) by subjecting 1-adamantylamine or a 1-adamantylammonium salt to catalytic methylation with methanol.

10. The process according to any of the preceding claims, the reaction with dimethyl sulfate in step A) taking place at a temperature of at least 40°C, more preferably of at least 80°C.

11. The process according to any of the preceding claims, the molar amount ratio of 1-adamantyldimethylamine to dimethyl sulfate in step A) being at least 2:1.

12. The process according to any of the preceding claims, step A) producing a methylation product which comprises at least 95%, more preferably at least 99%, more particularly at least 99.9%, by weight, of 1-adamantyltrimethylammonium sulfate, based on the total weight of 1-adamantyltrimethylammonium sulfate and 1-adamantyltrimethylammonium methylsulfate.

13. The process according to any of the preceding claims, a strongly basic anion exchanger being used in step B).

14. The process according to any of the preceding claims, step B) producing an aqueous 1-adamantyltrimethylammonium hydroxide solution which is optionally subjected to partial separation from water.

15. The process according to claim 14, the 1-adamantyltrimethylammonium hydroxide solution obtained, optionally after partial separation from water, having a 1-adamantyltrimethylammonium hydroxide content of at least 10%, preferably at least 15%, more particularly at least 18%, by weight, based on the total weight of the solution.

## Revendications

1. Procédé pour la préparation d'hydroxyde de 1-adamantyltriméthylammonium, dans lequel
A) on prépare de la 1-adamantyldiméthylamine et on la soumet à une transformation avec du diméthylsulfate, les deux groupes méthyle du diméthylsulfate réagissant essentiellement et du sulfate de 1-adamantyltriméthylammonium étant essentiellement obtenu, et
B) on soumet le sulfate de 1-adamantyltriméthylammonium obtenu dans l'étape A) à un échange d'anions sur un échangeur d'ions chargé d'ions OH.

2. Procédé selon la revendication 1, dans lequel, pour la préparation de la 1-adamantyldiméthylamine dans l'étape A), on soumet de la 1-adamantylamine ou un sel de 1-adamantylammonium à une diméthylation.

3. Procédé selon la revendication 2, dans lequel, pour la préparation de la 1-adamantyldiméthylamine, on part de chlorhydrate de 1-adamantylammonium.

4. Procédé selon la revendication 3, le chlorhydrate de 1-adamantylammonium étant d'abord transformé en 1-adamantylamine par transformation avec une base aqueuse.

5. Procédé selon l'une quelconque des revendications 1 à 4, dans lequel, pour la préparation de la 1-adamantyldiméthylamine dans l'étape A), on soumet du formaldéhyde à une amination réductrice avec de la 1-adamantylamine ou un sel de 1-adamantylammonium et avec de l'hydrogène en présence d'un catalyseur d'hydrogénation.

6. Procédé selon la revendication 5, la transformation ayant lieu dans un système qui présente deux phases liquides.

7. Procédé selon l'une quelconque des revendications 5 ou 6, dans lequel
a) on transforme du chlorhydrate de 1-adamantylammonium en 1-adamantylamine par traitement avec une solution aqueuse d'une base,
b) on soumet la 1-adamantylamine à une amination réductrice avec du formaldéhyde et de l'hydrogène en présence d'un catalyseur d'hydrogénation en 1-adamantyldiméthylamine,
c) on soumet la 1-adamantyldiméthylamine à une transformation avec du diméthylsulfate, les deux groupes méthyle du diméthylsulfate réagissant essentiellement et du sulfate de 1-adamantyltriméthylammonium étant essentiellement obtenu, et
d) on soumet le sulfate de 1-adamantyltriméthylammonium à un échange d'anions sur un échangeur d'ions chargé d'ions OH.

8. Procédé selon l'une quelconque des revendications 1 à 4, dans lequel, pour la préparation de la 1-adamantyldiméthylamine dans l'étape A), on soumet du formaldéhyde à une amination réductrice avec de la 1-adamantylamine ou un sel de 1-adamantylammonium en présence d'acide formique.

9. Procédé selon l'une quelconque des revendications 1 à 4, dans lequel, pour la préparation de la 1-adamantyldiméthylamine dans l'étape A), on soumet de la 1-adamantylamine ou un sel de 1-adamantylammonium à une méthylation catalytique avec du méthanol.

10. Procédé selon l'une quelconque des revendications précédentes, la transformation avec le diméthylsulfate dans l'étape A) étant réalisée à une température d'au moins 40°C, de manière particulièrement préférée d'au moins 80°C.

11. Procédé selon l'une quelconque des revendications précédentes, le rapport des quantités molaires de 1-adamantyldiméthylamine à diméthylsulfate dans l'étape A) valant au moins 2:1.

12. Procédé selon l'une quelconque des revendications précédentes, un produit de méthylation étant obtenu dans l'étape A), qui contient au moins 95% en poids, de manière particulièrement préférée au moins 99% en poids, en particulier au moins 99,9% en poids, de sulfate de 1-adamantyltriméthylammonium, par rapport au poids total de sulfate de 1-adamantyltriméthylammonium et de méthylsulfate de 1-adamantyltriméthylammonium.

13. Procédé selon l'une quelconque des revendications précédentes, un échangeur d'anions fortement basique étant utilisé dans l'étape B).

14. Procédé selon l'une quelconque des revendications précédentes, une solution aqueuse d'hydroxyde de 1-adamantyltriméthylammonium étant obtenue dans l'étape B), qui est le cas échéant soumise à une séparation partielle d'eau.

15. Procédé selon l'une quelconque des revendications 14, la solution d'hydroxyde de 1-adamantyltriméthylammonium obtenue, le cas échéant après une séparation partielle d'eau, présentant une teneur en hydroxyde de 1-adamantyltriméthylammonium d'au moins 10% en poids, de préférence d'au moins 15% en poids, en particulier d'au moins 18% en poids, par rapport au poids total de la solution.
